(19) Europäisches Patentamt
European Patent Office
Office européen des brevets

(11) **EP 2 114 360 B1**

(12) **EUROPEAN PATENT SPECIFICATION**

(45) Date of publication and mention of the grant of the patent:
**25.03.2015 Bulletin 2015/13**

(21) Application number: **08869798.2**

(22) Date of filing: **05.02.2008**

(51) Int Cl.:
*A61Q 5/12* (2006.01)          *A61K 8/46* (2006.01)
*A61K 8/49* (2006.01)          *A61Q 5/06* (2006.01)

(86) International application number:
**PCT/US2008/053014**

(87) International publication number:
**WO 2009/088520 (16.07.2009 Gazette 2009/29)**

(54) **HAIR CARE COMPOSITION**

HAARPFLEGEZUSAMMENSETZUNG

COMPOSITION POUR LE SOIN DES CHEVEUX

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.02.2007 US 702308**
**30.10.2007 US 978899**

(43) Date of publication of application:
**11.11.2009 Bulletin 2009/46**

(73) Proprietor: **The Procter & Gamble Company**
**Cincinnati, OH 45202 (US)**

(72) Inventors:
• **CARBALLADA, Jose, Antonio**
**Cincinnati**
**Ohio 45251 (US)**
• **NIJAKOWSKI, Timothy, Roy**
**Mason**
**Ohio 45040 (US)**

• **MURPHY, Bryan, Patrick**
**Loveland**
**Ohio 45140 (US)**
• **HUYGHUES-DESPOINTES, Alexis, M.J.A.**
**Cincinnati**
**Ohio 45201 (US)**
• **KALBFLEISCH, Axel**
**64295 Darmstadt (DE)**

(74) Representative: **Holmes, Rosalind**
**Procter & Gamble Service GmbH**
**Legal Innovation**
**Patent Department**
**Frankfurter Straße 145**
**61476 Kronberg im Taunus (DE)**

(56) References cited:
**EP-B- 1 159 318          WO-A-2005/023207**
**WO-A-2007/127065          US-A- 3 937 802**
**US-A- 5 362 486**

**Description**

FIELD OF THE INVENTION

The present invention relates to hair care compositions comprising a sulfopropylacrylate compound. The present invention further relates to a kit comprising a first composition comprising a sulfopropylacrylate compound and a second composition comprising an initiator.

BACKGROUND OF THE INVENTION

Hair style formation and retention traditionally has been accomplished by the use of styling products comprising polymers and other components that at least partially coat the hair and act on the surface of hair fibers. Several disadvantages are associated with this approach. Loss of hair style due to passage of time, elevated humidity, excessive motion, etc. may cause a consumer to feel the need to refresh a hair style throughout the day. This often requires application of additional styling product(s). The benefits of traditional styling products also may be diminished when applied to hair to which conditioning agents also have been applied. In addition, application of materials to the surface of hair may compromise the natural feel and appearance of the hair and result in a dull appearance and/or a stiff or otherwise unpleasant feel.

A need exists, therefore, to provide hair care compositions that avoid the aforementioned disadvantages, and that provide improved shape retention and style durability, with or without the use of a hair conditioning agent. Such compositions may be especially desirable for consumers seeking a hair style with increased volume.

SUMMARY OF THE INVENTION

Without wishing to be limited by theory, hair is comprised of an outer layer, or cuticle, which in turn comprises overlapping cuticle cells that serve to protect the inner hair shaft. The inner hair shaft, or cortex, comprises cortical cells. Both cuticle and cortical cells are made of proteins which are crosslinked and arranged in specific patterns. The proteins of the cuticle and cortical cells may play a role in controlling the mechanical properties of hair, for example, rigidity, strength, and elasticity. Applicants believe that selected monomers having a relatively low molecular weight are able to penetrate the cuticle and cortex and bind to both reduced keratinous proteins and to each other. Thus, rather than applying a pre-formed polymer to the outer layer of the hair, the present invention describes applying relatively small molecules that are able to penetrate into the internal region of the hair shaft, where the molecules may bond to the hair and/or to each other to form larger molecules. This, in turn, internally increases the rigidity of the hair, which allows style formation of increased volume and style retention for longer periods of time.

The present invention offers several advantages over other approaches. Because the complexes formed by the monomers with the keratinous proteins remain stable for an extended period of time, hair treated with the compositions of the present invention may continue to exhibit increased rigidity after multiple washings and/or wetting of the hair. The hair further exhibits fewer negative effects of moisture and/or humidity, such as frizziness or limpness. The hair also may be easier to style, may require application of less styling product, and/or may hold the style for longer periods of time.

The following describe some non-limiting embodiments of the present invention. According to a first embodiment of the present invention, a hair care composition is provided, comprising from 0.1% to 20% by weight of the total composition of a sulfopropylacrylate compound, wherein the sulfopropyl acrylate compound has a molecular weight of less than 500 grams/mole; and a dermatologically acceptable carrier; wherein the composition comprises from about 60% to about 99.9% by weight of the total composition of a dermatologically acceptable carrier.

According to yet another embodiment of the present invention, a kit is provided comprising a first composition according to the first embodiment; and a second composition comprising an initiator; wherein the first composition and the second composition are separately packaged within the kit.

According to an unclaimed embodiment of the present invention, an article of commerce is provided comprising a first composition according to the first embodiment; a second composition comprising an initiator, and advertisement material pertaining to the compositions. The advertisement material may comprise images comparing the appearance of a person prior to use of the compositions to the appearance of the same person after use of the composition.

According to another unclaimed embodiment of the present invention, a method of marketing a kit comprising a first composition according to the first embodiment; and a second composition comprising an initiator, wherein the method of marketing comprises the step of making available to a consumer the kit, and providing a communication to the consumer that the compositions may provide one or more benefits to the hair, including but not limited to increased style retention, increased style durability, increased appearance of volume, increased resistance to moisture, and combinations thereof.

DETAILED DESCRIPTION OF THE INVENTION

In all embodiments of the present invention, all percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or byproducts that may be included in commercially available materials, unless otherwise specified.

"Hair," as used herein, means hair on the human head and scalp. "Hair shaft" means an individual hair, and may be used interchangeably with the term "hair."

"Internal region of the hair shaft," as used herein, means any non-surface portion of the hair shaft, including the inner portion of the cuticle. "Non-surface portion" may be understood to mean that portion of the hair that is not in direct contact with the outside environment.

"Proximal to the scalp," as used herein, means that portion of an extended, or substantially straightened, hair shaft that is closer in distance to the scalp than to the end of the hair. Thus, about 50% of the hair would be considered proximal to the scalp, and about 50% of the hair would be distal to the scalp. "x cm proximal to the scalp" means a distance "x" along the hair, with one endpoint being on or directly adjacent to the scalp, and the second endpoint being measured "x" centimeters along the length of the extended or substantially straightened hair.

"Dermatologically-acceptable carrier," as used herein, that the compositions or components described are suitable for use in contact with human keratinous tissue without undue toxicity, incompatibility, instability, allergic response, and the like.

"Derivatives," as used herein, includes but is not limited to, amide, ether, ester, amino, carboxyl, acetyl, and/or alcohol derivatives of a given compound.

"Monomer," as used herein, means a discrete, non-polymerized chemical moiety capable of undergoing polymerization in the presence of an initiator.

"Ethylenic monomer," as used herein, means a chemical species that contains an olefenic carbon-carbon double bond (C=C) and is capable of undergoing polymerization in the presence of an initiator.

"Multivalent cation," as used herein, means an element having a net ionic charge of from +2 to +7.

"Chemically modify," or grammatical equivalents thereof, as used herein, means that a chemical moiety such as monomer and/or crosslinker, stably affixes to a second chemical moiety, for example, a keratin protein, another component of hair, and/or another monomer or crosslinker.

"Stably affix" is understood to include both covalent and non-covalent forms of chemical bonds that once formed, remain unchanged through wetting, washing, styling and other types of hair treatment. In general, stably affixed chemical moieties may not be removed from the hair without damaging or substantially destroying the hair.

"Bending rigidity," as used herein, is determined by the Kato Single Fiber Bending Method. One example of determining bending rigidity is as follows: Select 6 individual hairs from an area to which the composition(s) of the present invention will be applied. Mount a brass ferrule to one end of an individual fiber using a RS Components Press - Sock No. 622-032. Cut the hairs to a length of 55mm from the base of the ferrule. Using a KES-FB2-SH Single Fiber Bending Tester System (Kato Tech Co., LTD) run by KES-FB system measurement program version 6.35WE, run "Single Hair Fiber Bending Method" using the following settings: Sample = Hair; Sensitivity = 2.5; Count = 1; Curvature = 2.5; Repetition = 1. Load the sample as follows: Lock the back chuck, set check switch on OSC and confirm a meter reading of +10V. Settings: Sensitivity (initially) = 0.08 gf/10V; speed switch = 1; speed dial = 2. Place the hair sample end without the brass ferrule in the rear chuck, centered vertically. Very lightly tighten rear chuck with knob on side. Pull fiber through the front chuck, ensuring fiber is straight, not taut, and not loose. Tighten chuck with knob. Unlock chuck holder and shut the enclosure. Perform measurement as follows: Set check knob to BAL and obtain a reading of 0V on the voltmeter. Set check knob to MES and obtain a reading of 0V on the voltmeter. Measure each hair 3 times, reloading the sample prior to each evaluation, to obtain a total of 18 readings for the set of six hair samples. Average the 18 readings to obtain a measurement of the "bending moment" in gf cm/fiber, where "gf" means grams of force, for the set of samples. (A typical bending moment value is approximately 0.2 gf cm/fiber.) Repeat the procedure for the same set of samples after application, as described herein, of the composition(s) of the present invention. The bending rigidity is increased if the average bending moment of the same set of samples after treatment described in the present invention is at least 20% greater than the average bending moment of the initial measurement.

"Curl retention" may be measured as follows: Using a swatch of hair weighing approximately 1g and having a length of about 10 inches, thoroughly wet the hair with water and comb sufficiently to detangle. Affix the top portion of the hair, for example with a rubber band, to a ceramic rod having a length of 6 inches and a diameter of 0.5 inches. Wrap the hair around the rod in a spiral fashion with substantially no overlap of hair per spiral and with no space between each spiral. Stop wrapping around the rod when approximately 2 inches of hair are remaining, which are then enclosed with folded end papers as one of skill in the art would understand to apply to permed hair. Continue curling the wrapped last two inches around the ceramic rod and attach the bottom portion of the hair to the rod using adhesive tape which contacts the rod and endpaper only. Allow the hair to set in this curled position for 8 - 20 hours or until dry. Remove the hair swatch from the rod and hang vertically. Optionally, the hair may be hung in an environment having a temperature of about 32°C and about 80% relative humidity. Measure the total length of the curled hair from top to bottom, and record as the length at time zero ($length_{(time\ 0)}$). Repeat the measurement after 60 minutes and record as the total length of the hair at time 60 ($length_{(time\ 60)}$). Calculate the curl retention number (CRN) as:

$$\text{Curl Retention Number}_{(time\ 60)} = [\text{Length}_{(time\ 60)} - \text{Length}_{(time\ 0)}] / [\text{Length}_{(time\ 0)}]$$

Repeat the method with four additional swatches, and average the five measurements of the difference in total length to obtain the average Curl Retention Number. "Increased curl retention" means that the average curl retention number of a set of five swatches of hair to which the composition(s) of the present invention have been applied is at least 20% less than the average curl retention number of a control set of five swatches, treated according to the same method but to which no composition(s) of the present invention have been applied.

"Increased style retention and/or durability," as used herein, means that the hair style formed, shaped, and/or obtained after application of the composition of the present invention to the hair is maintained for a longer period of time relative to hair of the same being to which no composition has been applied.

"Increased appearance of volume," as used herein, means that the hair exhibits a visually noticeable greater volume, i.e., distance between the scalp and the outermost layer of hair style and/or distance between individual hairs, after application of a composition of the present invention relative to before a composition was applied.

"Increased resistance to moisture," as used herein, means that after application of a composition of the present invention, the hair fails to exhibit visually noticeable effects, such as loss of volume, loss of style, increase in frizz, etc. upon exposure to water vapor (i.e., relative humidity greater than 50%) relative to before a composition has been applied.

"Reserve alkalinity," as used herein, means the relative strength and apparent concentration of the base used to adjust the pH, measured as described in ASTM D 1121, wherein the base of the present invention is substituted for the antifreeze used in the method.

"Kit," as used herein, means a packaging unit comprising a first composition and a separately packaged second composition, as described herein.

"Separately packaged," as used herein, means any form of packaging that prevents a first composition from coming into physical contact, or admixing, with a second composition. "Separately packaged" may mean that the individual compositions are packaged in separate containers, or alternatively in a single container partitioned such that the compositions are not in physical contact.

"Implement," as used herein, means a device used to facilitate application of a composition to the hair and/or manipulation of the hair. Examples of implements include, but are not limited to, a comb, a means for directed delivery (e.g., an applicator or tube), a covering for the hair (e.g., plastic bag, shower cap, etc.), and combinations thereof.

"Energy delivery device," as used herein, means any device used to deliver energy to keratinous tissue, including the hair and scalp. "Delivery of energy," means that the surface of the keratinous tissue is exposed to the energy emanating from the energy delivery device, where it may penetrate to the desired layers of the tissue, including the hair shaft and/or hair follicle. Energy includes but is not limited to energy in the form of light, heat, sound (including ultrasonic waves), electrical energy, magnetic energy, electromagnetic energy (including radiofrequency waves and microwaves), and combinations thereof.

I. Compositions

The present invention comprises a composition comprising a sulfopropylacrylate compound. When referring to an embodiment that contemplates more than one composition, the composition comprising a sulfopropylacrylate compound may be referred to as a "first composition." The composition may have a pH of 7.0 and below, alternatively of from 2.0 to 7.0, and alternatively of from 3.0 to 6.0.

"Sulfopropylacrylate compound," as used herein, is understood to include derivatives, salts and/or isomers of sulfopropylacrylate. The sulfopropylacrylate compounds of the present invention are understood to be in a non-polymerized form, and do not include polymers of sulfopropyl(meth)acrylate. Sulfopropylacrylate compounds also may be known as sulfopropyl acrylate esters; sulfoalkylpropenoate; acrylic acid propyl ester sulfonates; propenoic acid sulfoalkyl ester; and/or sulfoalkylpropenoic acid ester. The composition may comprise from 1% to 15%, alternatively from 5% to 10%, alternatively from 0.1% to 10%, and alternatively from 10% to 20% of a sulfopropylacrylate compound.

The sulfopropylacrylate compound is of a size suitable to penetrate the hair shaft, and have a molecular weight of 500 g/mole or less, alternatively from 100 g/mole to 500 g/mole, alternatively from 100 g/mole to 400 g/mole, and alternatively from 200 g/mole to 400 g/mole, where "g/mole" means grams per mole of a compound.

The composition further may comprise at least one ethylenic monomer having a size suitable to penetrate the hair shaft and a molecular weight of 500 g/mole or less, alternatively from 50 g/mole to 500 g/mole, alternatively from 75 g/mole to 400 g/mole, and alternatively from 100 g/mole to 400 g/mole. Examples of ethylenic monomers suitable for use in the first composition of the present invention include, but are not limited to mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, and salts, isomers, derivatives and mixtures thereof. The composition may comprise from 0.1% to 20%, alternatively from 1% to 15%, alternatively from 5% to 10%, alternatively from 0.1% to 10%, and alternatively from 10% to 20%, of an ethylenic monomer.

In one embodiment, the ratio of the weight percentage of the sulfopropylacrylate compound to the weight percentage of the monomer is from 1:12 to 12:1, alternatively from 1:10 to 10:1, alternatively from 1:5 to 5:1, alternatively from 12:1 to 1:1, alternatively from 10:1 to 1:1 and alternatively from 5:1 to 1:1.

The first composition further may comprise at least one crosslinker having a molecular weight of a size suitable to penetrate the hair shaft and a molecular weight of 500 g/mole or less, alternatively from 100 g/mole to 500 g/mole, alternatively from 100 g/mole to 400 g/mole, and alternatively from 200 g/mol to 400 g/mole. Examples of crosslinkers suitable for use in the first composition of the present invention include, but are not limited to, 1,4-bisacryloylpiperazine, methylenebisacrylamide, ethylenebisacrylamide, divinylbenzene, poly-ethyleneglycol di(meth)acrylate, ethylene glycol di(meth)acrylate, 1,3-butanediol di(meth)acrylate, 1,4-butanediol di(meth)acrylate, neopentyl glycol di(meth)acrylate, and mixtures thereof.

In one embodiment, the ratio of the weight percentage of the sulfopropylacrylate compound to the weight percentage of the crosslinker is from 50:1 to 10:1, alternatively from 40:1 to 10:1, and alternatively from 20:1 to 10:1.

The first composition further may comprise from 0.01% to 1% of at least one organic or inorganic catalyst. Non-limiting examples of suitable organic catalysts include 2-pyrolidinoethanol, 1-piperidine-ethanol, 4-methylmorpholine, 2-morpholinoethanol, tetramethylethylenediamine, and mixtures thereof. Non-limiting examples of suitable inorganic catalysts include salts and/or hydrates of cerium(IV), cobalt(III), manganese(III), iron(III), nickel(II), copper(II), and mixtures thereof.

The present invention further may comprise, in some embodiments, a second composition comprising an initiator, useful for promoting binding of an ethylenic monomer and/or crosslinker to the keratin and/or to another monomer. The second composition may comprise from 0.001% to 5%, alternatively from 0.01% to 1%, and alternatively from 0.1% to 1%, of an initiator. Examples of suitable classes of initiators include, but are not limited to, peroxidisulfates, peroxides, peracids, phosphates, manganates, borates, bis-alkyamidines, sulfites, peroxyesters, bis-cyanocarboxylic acids, alphaamino acetic acids, and mixtures thereof. Non-limiting examples of suitable initiators include sodium peroxydisulfate, 2,2'-azobis(2-methylpropionamidine)dihydrochloride, 2,2'-azobisisobutyronitrile, benzoyl peroxide, peracetic acid, ammonium cerium(IV) nitrate, and mixtures thereof. The second composition further may have a pH of above 7.0, alternatively of from 7.0 to 12.0, alternatively of from 8.0 to 12.0, and alternatively of from 9.0 to 11.0. In an alternative embodiment, the second composition has a pH of 7.0 and below.

The present invention may comprise a third composition comprising a base, which is packaged separately from the first and the second composition, and which is useful for adjusting the pH of one, or a combination of any, of the compositions disclosed herein. The third, or pH adjusting, composition may have a pH of above 7.0, alternatively of from 7.0 to 12.0, alternatively of from 8.0 to 12.0, and alternatively of from 9.0 to 11.0. Additionally or alternatively, the third composition has a reserve alkalinity from 1 to 40, and alternatively from 10 to 30. Non-limiting examples of classes of suitable bases include, but are not limited to ammonium salts, amines, hydroxides, metasilicates, and mixtures thereof. Non-limiting examples of suitable bases include sodium hydroxide, potassium hydroxide, sodium metasilicate, ammonium hydroxide, ethanolamine, aminomethylpropanol, ammonium carbonate, and mixtures thereof.

The third composition further may comprise from 0.1% to 10%, and alternatively from 0.5% to 5%, of at least one organic or inorganic salt. Examples of organic salts include, but are not limited to, salts formed by reacting at least one anion chosen from phosphates, borates, silicates, bicarbonates, carbonates, chlorates, nitrates, halides (including, but not limited to, chlorides), and/or sulfonates, with at least one cation chosen from potassium, sodium, strontium, cadmium, calcium, ammonium (such as tetraalkylammonium and arylammonium), phosphonium, barium, lithium, and/or magnesium. Non-limiting examples of suitable organic salts include sodium monobutyl and dibutyl phosphates and sodium monoethyl and diethyl phosphates. In one embodiment, the salt comprises an inorganic cation. In one embodiment, the inorganic cation is a multivalent cation selected from the group consisting of magnesium, calcium, strontium, barium, copper, zinc, iron, nickel, cobalt, manganese, aluminum, silver, lanthanum, and complexes and mixtures thereof.

The present invention may comprise a fourth composition, useful for penetrating the hair shaft and reducing disulfide bonds. The fourth, or reducing, composition, comprises from 1% to 12%, alternatively from 4% to 10%, and alternatively from 8% to 10%, of a reducing agent. Examples of suitable reducing agents include, but are not limited to, sodium thioglycolate, anhydrous sodium thiosulfate, powdered sodium metabisulfite, thiourea, ammonium sulfite, thioglycolic acid, thiolactic acid, ammonium thiolactate, glyceryl monothioglycolate, ammonium thioglycolate, thioglycerol, 2,5-dihydroxybenzoic acid, diammonium dithioglycolate, strontium thioglycolate, calcium thioglycolate, zinc formosulfoxylate, isooctyl thioglycolate, dl-cysteine, monoethanolamine thioglycolate, phosphines, and mixtures thereof.

Dermatologically Acceptable Carrier

The compositions of the present invention may comprise from 60% to 99.9% by weight of the total composition, alternatively from 70% to 95%, by weight of the total composition and alternatively from 80% to 90%, by weight of the total composition of a dermatologically acceptable carrier. Carriers suitable for use with the composition(s) of the present invention include, for example, those used in the formulation of hair sprays, mousses, tonics, gels, and leave-on conditioners. The carrier may comprise water; organic oils; silicones such as volatile silicones, amino or non-amino silicone gums or oils, and mixtures thereof; mineral oils; plant oils such as olive oil, castor oil, rapeseed oil, coconut oil, wheatgerm oil, sweet almond oil, avocado oil, macadamia oil, apricot oil, safflower oil, candlenut oil, false flax oil, tamanu oil, lemon oil and mixtures thereof; waxes; and organic compounds such as $C_2$-$C_{10}$ alkanes, acetone, methyl ethyl ketone, volatile organic $C_1$-$C_{12}$ alcohols, esters of $C_1$-$C_{20}$ acids and of $C_1$-$C_8$ alcohols such as methyl acetate, butyl acetate, ethyl acetate, and isopropyl myristate, dimethoxyethane, diethoxyethane, $C_{10}$-$C_{30}$ fatty alcohols such as lauryl alcohol, cetyl alcohol, stearyl alcohol, and behenyl alcohol; $C_{10}$-$C_{30}$ fatty acids such as lauric acid and stearic acid; $C_{10}$-$C_{30}$ fatty amides such as lauric diethanolamide; $C_{10}$-$C_{30}$ fatty alkyl esters such as $C_{10}$-$C_{30}$ fatty alkyl benzoates; hydroxypropylcellulose, and mixtures thereof. In one embodiment, the carrier comprises water, fatty alcohols, volatile organic alcohols, and mixtures thereof.

Optional Components

The composition(s) of the present invention may further comprise one or more optional components known or otherwise effective for use in hair care or personal care products, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004, both of which are incorporated by reference herein in their entirety. Some non-limiting examples of such optional components are disclosed below, and include plasticizers, surfactants (which may be anionic, cationic, amphoteric or nonionic), neutralizing agents, propellants, hair conditioning agents (e.g., silicone fluids, fatty esters, fatty alcohols, long chain hydrocarbons, cationic surfactants, etc.), emollients, lubricants and penetrants such as various lanolin compounds, vitamins, proteins, preservatives, dyes, tints, bleaches, reducing agents and other colorants, sunscreens, thickening agents (e.g., polymeric thickeners, such as xanthan gum), physiologically active compounds for treating the hair or skin (e.g., anti-dandruff actives, hair growth actives), non-polymeric thickeners including clays, and perfume.
The composition(s) of the present invention further may comprise from 0.1% to 10%, and alternatively from 0.2% to 5.0%, of a gelling agent to help provide the desired viscosity to the composition(s). Non-limiting examples of suitable optional gelling agents include crosslinked carboxylic acid polymers; unneutralized crosslinked carboxylic acid polymers; unneutralized modified crosslinked carboxylic acid polymers; crosslinked ethylene/maleic anhydride copolymers; unneutralized crosslinked ethylene/maleic anhydride copolymers (e.g., EMA 81 commercially available from Monsanto); unneutralized crosslinked allyl ether/acrylate copolymers (e.g., Salcare™ SC90 commercially available from Allied Colloids); unneutralized crosslinked copolymers of sodium polyacrylate, mineral oil, and PEG-1 trideceth-6 (e.g., Salcare™ SC91 commercially available from Allied Colloids); unneutralized crosslinked copolymers of methyl vinyl ether and maleic anhydride (e.g., Stabileze™ QM-PVM/MA copolymer commercially available from International Specialty Products); hydrophobically modified nonionic cellulose polymers; hydrophobically modified ethoxylate urethane polymers (e.g., Ucare™ Polyphobe Series of alkali swellable polymers commercially available from Union Carbide); and combinations thereof. In this context, the term "unneutralized" means that the optional polymer and copolymer gelling agent materials contain unneutralized acid monomers. Preferred gelling agents include water-soluble unneutralized crosslinked ethylene/maleic anhydride copolymers, water-soluble unneutralized crosslinked carboxylic acid polymers, water-soluble hydrophobically modified nonionic cellulose polymers and surfactant/fatty alcohol gel networks such as those suitable for use in hair conditioning products.

II. Kit

The present invention further describes a kit comprising a first composition and a second composition as described herein. The first and the second composition may be packaged in separate containers within the kit, and alternatively may be packaged in a single container which is capable of preventing admixing of the two compositions. The packaging may be of a size suitable for a single application, or unit dose, of the first and/or second composition. The kit may comprise a number of unit doses suitable for an indicated treatment regimen.

The kit further may comprise at least one additional composition selected from the group consisting of a pH adjustor, a reducing composition, and combinations thereof. In one embodiment, the pH adjustor has a reserve alkalinity as described herein and packaged in a container having a volume, such that when the contents of the container are mixed with the first and/or the second composition, the pH of the resulting mixture of compositions is greater than 7.0.

The kit further may comprise at least one additional component selected from the group consisting of a shampoo, a conditioner, a neutralizer, a colorant, a styling aid such as a gel, a mousse, a pomade, etc., an implement, an energy delivery device, instructions for complying with a treatment regimen, and combinations thereof. Examples of energy delivery devices include, but are not limited to light sources, including UV, visible light and infrared light, temperature change elements, hair dryers, heaters such as irons and heated curlers, ultrasonic devices, etc.

The kit further may comprise instructions for complying with a hair treatment regimen. The treatment regimen may comprise one or methods of use described herein, and may be directed toward treatment by a professional stylist or toward treatment by a consumer who is not a professionally-trained stylist.

III. Article of Commerce

An unclaimed embodiment describes an article of commerce comprising a first composition and/or a second composition, as described herein, and a communication pertaining to the compositions. The communication may be printed material attached directly or indirectly to packaging, for example to a kit, that contains the compositions. Alternatively, the communication may be placed directly or indirectly near at least one composition. Alternatively, the communication may be an electronic or a broadcast message that is associated with the applicator and/or the composition. The communication may comprise images comparing the appearance of a person prior to use of the compositions to the appearance of the same person after use of the composition. One example of a suitable communication would be one directing a consumer having thin and/or limp hair to apply the composition(s) to the hair to increase the volume of hair and/or to create fuller looking hair for an extended period of time. Another example of a suitable communication would be one directing a professional hair stylist to apply the composition to clients desiring a longer lasting treatment to increase the volume of hair, the ease of styling, the retention of a style, etc.

IV. Method of Marketing

An unclaimed embodiment comprises a method of marketing a kit comprising a first composition and a second composition as described herein, wherein the method of marketing comprises the step of making available to a consumer the kit, and providing a communication to the consumer that the compositions may provide one or more benefits to the hair, including but not limited to increased appearance of hair volume, increased resistance of the hair to moisture, increased ease of styling, and/or increased retention of a hair style. Examples include all day hold of style, excellent curl definition, increased body and/or fullness, the ability to curl straight hair, and/or the ability to straighten curly hair.

Examples

I. The following describes one non-limiting example of a method of using the compositions of the present invention to increase the shape retention and the durability of mammalian hair:

Wash the hair or portion of hair to be treated with a cleansing shampoo. Thoroughly rinse and towel dry the hair, and comb the hair sufficiently to detangle. Apply a sufficient amount (estimated based upon the amount of hair to be treated), of a fourth composition, as described herein, to penetrate the hair shaft and to cleave disulfide bonds. Work the composition into hair with gentle messaging, avoiding pulling hair and avoiding contact of the composition with the skin and/or scalp and eyes. Optionally, the lotion may be evenly distributed by using a comb or other suitable implement. Allow the composition to remain on the hair, uncovered, for a period of time suitable to substantially cleave the disulfide bonds, as would be known by one of skill in the art such as a professional stylist. One example of a suitable time is 10 minutes at a temperature of 35°C ± 5°C. Rinse the composition from the hair with warm water. Pat hair lightly with a towel until hair is damp and not dripping wet. Do not blow dry or comb hair.

Mix together by stirring or vigorously shaking, for about 30 seconds, about 50 ml of a first composition (Composition I - described below), about 1.0 ml of a second composition (Composition II - described below), and about 49 ml of a pH adjusting composition (Composition III - described below). Apply a suitable amount, as described above in this example, of the mixture to the damp hair. Gently massage the mixture into the hair, avoiding contact of the composition with the skin and/or scalp and eyes. Allow the composition to remain on the hair, uncovered, for about 30 minutes at 35°C $\pm$ 5°C. Gently wash the hair (preferably with clarifying shampoo) and rinse thoroughly with water. Repeat the washing and rinsing once. Optionally, a conditioner may be applied to the hair. The hair may further be dried and styled as desired.

Composition I

| | Example 1 wt. % | Example 2 wt. % | Example 3 wt. % | Example 4 wt. % | Example 5 wt. % | Example 6 wt. % | Example 7 not claimed wt. % |
|---|---|---|---|---|---|---|---|
| Ingredients: | | | | | | | |
| 1 Purified Water | qs | qs | qs | qs | qs | qs | qs |
| 2 Cetyl Alcohol | 2.25 | - | 4.5 | 4.5 | - | 2.25 | - |
| 3 Stearyl Alcohol | 2.25 | - | 4.5 | 4.5 | - | 2.25 | - |
| 4 Hydroxypropylmethyl cellulose | - | 0.8 | - | 0.8 | 0.4 | - | 1.0 |
| 5 Guar hydroxypropyltrimonium chloride | - | 0.8 | - | - | 0.8 | - | - |
| 6 Polyquaterium-10 | - | - | - | - | 2.5 | 1 | 1.5 |
| 7 Ceteareth 15 | 1.5 | - | 1.5 | 1.5 | - | 1.5 | - |
| 8 Tetrasodium EDTA | 0.1 | 0.1 | 0.1 | 0.1 | 0.1 | 0.1 | 0.05 |
| 9 Phenoxyethanol | 0.3 | - | 0.3 | 0.3 | - | 0.3 | 0.25 |
| 10 Sodium Benzoate | 0.25 | 0.25 | 0.25 | 0.25 | 0.25 | 0.25 | 0.125 |
| 11 4-(2-Hydroxyethyl)-morpholine | - | 0.08 | - | - | - | - | - |
| 12 1,2-Octanediol | - | - | 2 | - | - | - | 1 |
| 13 Isopropyl alcohol | - | - | 10 | - | - | - | - |
| 14 Eldew™ SL-205[1] | - | - | 2 | - | - | 2 | 1 |
| 15 Methyl Paraben | - | 0.4 | - | - | 0.4 | - | - |
| Monomer Actives: | | | | | | | |
| 16 Sulfopropylacrylate potassium salt | 9.6 | 15 | 15 | 16 | 16 | 0.1 | 1 |
| 17 alpha-methylene-gamma-buyrolactone | 0.8 | - | 4 | - | 0.8 | - | 0.8 |
| 18 Bisacryoylpiperizine | - | 1.5 | 1 | - | - | - | - |
| 19 Methyl 2-acetamidoacrylate | - | 3.5 | - | - | - | - | - |
| Fragrance: | | | | | | | |
| 20 Nourrissant 124 | - | 0.25 | 0.25 | 0.25 | 0.25 | 0.25 | 0.15 |

[1] Isopropyl lauryl sarcosinate (Ajinomoto)

Mix the ingredients as follows, with continuous stirring. Add water to a suitable mixing vessel. Heat to about 80°C. Add ingredients 4, 5, 6, 8, and 10 and stir until dissolved. Add ingredients 2, 3, and 7, and allow temperature to again reach about 80°C. Add ingredients 9 and 11-15. Maintain temperature at about 80°C for about 30 minutes. Cool to about 30°C. Add ingredients 16-20 and continue to stir until dissolved.

Composition II

| Ingredients: | Example 1 wt. % | Example 2 wt. % | Example 3 wt. % | Example 4 wt. % | Example 5 wt. % | Example 6 wt. % | Example 7 not claimed wt. % |
|---|---|---|---|---|---|---|---|
| 21 Sodium Persulfate | 100 | - | 90 | 10 | 50 | 100 | 100 |
| 22 2,2-Azobis(2-methylpropionamidine) dihydrochloride | - | 100 | - | 90 | 50 | - | - |
| 23 Copper (III) Nitrate | - | - | 10 | - | - | - | - |

Combine all ingredients and mix well.

Composition III

| | | Example 1 | Example 2 | Example 3 | Example 4 | Example 5 | Example 6 | Example 7 not claimed |
|---|---|---|---|---|---|---|---|---|
| | Ingredients: | wt. % | wt. % | wt. % | wt. % | wt. % | wt. % | wt. % |
| 24 | Purified Water | qs | qs | qs | qs | qs | qs | qs |
| 25 | Ammonium Hydroxide (5N standard) | 8 | - | - | 10 | 10 | - | 10 |
| 26 | Sodium Hydroxide (1N standard) | - | 10 | - | - | - | 1 | - |
| 27 | Sodium Metasilicate | - | - | 10 | - | - | - | - |
| 28 | Barium Chloride | - | - | - | - | 8 | - | - |
| 29 | Strontium Nitrate | - | 8 | - | - | - | - | - |
| 30 | Strontium Chloride | | | - | - | - | - | 4.2 |
| 31 | Lanthanum Nitrate | | | 2 | | | | - |

Add water to a suitable mixing vessel. Add ingredients 25-30 and continue to stir until dissolved.

**Claims**

1. A hair care composition comprising from 0.1% to 20%, by weight of the total composition, of a sulfopropylacrylate compound, wherein the sulfopropylacrylate compound has a molecular weight of less than 500 grams/mole; and a dermatologically acceptable carrier; wherein the composition comprises from 60% to 99.9% by weight of the total composition of a dermatologically acceptable carrier.

2. The composition of claim 1, further comprising from 0.1 % to 20%, by weight of the total composition, of at least one additional compound selected from the group consisting of an ethylenic monomer, a crosslinker, and mixtures thereof, wherein the additional compound has a molecular weight of less than 500 g/mole.

3. The composition of claim 1, further comprising from 0.1% to 20%, by weight of the total composition, each of an ethylenic monomer and a crosslinker, wherein the ethylenic monomer and the crosslinker each have a molecular weight of less than 500 g/mole.

4. The composition of claim 2, wherein the ethylenic monomer is selected from the group consisting of mesaconic acid, tert-2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, and mixtures thereof.

5. The composition of claim 2, wherein the crosslinker is selected from the group consisting of 1,4-bisacryloylpiperazine, methylenebisacrylamide, ethylenebisacrylamide, divinylbenzene, poly-ethyleneglycol di(meth)acrylate, ethylene glycol di(meth)acrylate, 1,3-butanediol di(meth)acrylate, 1,4-butanediol di(meth)acrylate, neopentyl glycol di(meth)acrylate, or mixtures thereof.

**6.** The composition of claim 2, wherein the second compound is an ethylenic monomer, and wherein the ratio of the weight percentage of the sulfopropyl acrylate compound to the weight percentage of ethylenic monomer is from 1:12 to 12:1.

**7.** The composition of claim 2, wherein the second compound is a crosslinker, and wherein the ratio of the weight percentage of the sulfopropylacrylate compoundto the weight percentage of cross-linker is from 50:1 to 10:1.

**8.** The composition of claim 1, wherein the pH of the composition is 7.0 and below.

**9.** The composition of claim 1, wherein the composition comprises from 1% to 15%, alternatively from 5% to 10%, alternatively from 0.1% to 10%, and alternatively from 10% to 20% by weight of the total composition of a sulfopropylacrylate compound.

**10.** A kit comprising:

a) a first composition comprising from 0.1% to 20%, by weight of the total composition, of a sulfopropylacrylate compound, wherein the sulfopropylacrylate compound has a molecular weight of less than 500 grams/mole; and a dermatologically acceptable carrier; wherein the composition comprises from 60% to 99.9% by weight of the total composition of a dermatologically acceptable carrier; and
b) a second composition comprising an initiator;

wherein the first composition and the second composition are separately packaged.

**11.** The kit according to claim 10, wherein the second composition comprises from 0.001% to 5%, alternatively from 0.01% to 1%, alternatively from 0.1% to 1% by weight of the total composition of an initiator.

**12.** The kit according to any of claims 10 and 11, wherein the initiator is selected from: peroxidisulfates, peroxides, peracids, phosphates, manganates, borates, bis-alkyamidines, sulfites, peroxyesters, bis-cyanocarboxylic acids, alpha-amino acetic acids, and mixtures thereof.

**13.** The kit according to any of claims 10 to 11, wherein the second composition has a pH of above 7.0, alternatively of from 7.0 to 12.0, alternatively of from 8.0 to 12.0, alternatively of from 9.0 to 11.0.

**14.** The kit according to any of claims 10 to 13, wherein the kit further comprises an reducing composition, wherein the reducing composition comprises from 1% to 12%, alternatively from 4% to 10%, alternatively from 8% to 10%, by weight of the total composition of a reducing agent.

**15.** The kit according to claim 14, wherein the reducing agents is selected from sodium thioglycolate, anhydrous sodium thiosulfate, powdered sodium metabisulfite, thiourea, ammonium sulfite, thioglycolic acid, thiolactic acid, ammonium thiolactate, glyceryl monothioglycolate, ammonium thioglycolate, thioglycerol, 2,5-dihydroxybenzoic acid, diammonium dithioglycolate, strontium thioglycolate, calcium thioglycolate, zinc formosulfoxylate, isooctyl thioglycolate, dl-cysteine, monoethanolamine thioglycolate, phosphines, and mixtures thereof.

**Patentansprüche**

**1.** Haarpflegezusammensetzung, umfassend von 0,1 Gew.-% bis 20 Gew.-% einer Sulfopropylacrylatzusammensetzung bezogen auf die Gesamtzusammensetzung, wobei die Sulfopropylacrylatzusammensetzung ein Molekulargewicht von weniger als 500 Gramm/Mol aufweist; und einen dermatologisch akzeptablen Träger; wobei die Zusammensetzung von 60 Gew.-% bis 99,9 Gew.-% eines dermatologisch akzeptablen Trägers bezogen auf die Gesamtzusammensetzung umfasst.

**2.** Zusammensetzung nach Anspruch 1, ferner umfassend von 0,1 Gew.-% bis 20 Gew.-%, bezogen auf die Gesamtzusammensetzung, mindestens einer zusätzlichen Verbindung ausgewählt aus der Gruppe bestehend aus einem ethylenischen Monomer, einem Vernetzungsmittel und Mischungen davon, wobei die zusätzliche Verbindung ein Molekulargewicht von weniger als 500 g/Mol aufweist.

**3.** Zusammensetzung nach Anspruch 1, ferner umfassend von 0,1 Gew.-% bis 20 Gew.-% von jeweils einem ethylenischen Monomer und einem Vernetzungsmittel bezogen auf die Gesamtzusammensetzung, wobei das ethylenische Monomer und das Vernetzungsmittel jeweils ein Molekulargewicht von weniger als 500 g/Mol aufweisen.

4. Zusammensetzung nach Anspruch 2, wobei das ethylenische Monomer ausgewählt ist aus der Gruppe bestehend aus Mesaconsäure, tert-2-Pentansäure, Tiglinsäure, Tiglinsäureestern, Furan-3-acrylsäure, 2-Acrylamido-2-methyl-1-propansul-fonsäure, Maleinamidsäure, 3-Aminokrotonsäure, Krotonsäureestem, Itaconsäureanhydrid, Trimethylsilylacrylat, Poly(ethylenglycol)acrylaten, N-vinylacetamid, 2-Acetamidoacrylsäure, Vinylsulfonsäure, Tetralrydrofurfurylacrylat, N-methyl-N-vinylacetamid, Vinylpropionat, Vinylanisol, Vinylkrotonsäure, Methyl-3-hydroxy-2-methylenbutyrat und Mischungen davon.

5. Zusammensetzung nach Anspruch 2, wobei das Vernetzungsmittel ausgewählt ist aus der Gruppe bestehend aus 1,4-Bisacryloylpiperazin, Methylenbisacrylamid, Ethylenbisacrylamid, Divinylbenzol, Polyethylenglycoldi(meth)acrylat, Ethylenglycoldi(meth)acrylat, 1,3-Butandioldi(meth)acrylat, 1,4-Butandioldi(meth)acrylat, Neopentylglycoldi(meth)acrylat und Mischungen davon.

6. Zusammensetzung nach Anspruch 2, wobei es sich bei der zweiten Verbindung um ein ethylenisches Monomer handelt, und wobei das Verhältnis des Gewichtsprozentsatzes der Sulfopropylacrylatverbindung zum Gewichtsprozentsatz des ethylenischen Monomers von 1:12 bis 12:1 beträgt.

7. Zusammensetzung nach Anspruch 2, wobei es sich bei der zweiten Verbindung um ein Vernetzungsmittel handelt, und wobei das Verhältnis des Gewichtsprozentsatzes der Sulfopropylacrylatverbindung zum Gewichtsprozentsatz des Vernetzungsmittels von 50:1 bis 10:1 beträgt.

8. Zusammensetzung nach Anspruch 1, wobei der pH-Wert der Zusammensetzung 7,0 und weniger beträgt.

9. Zusammensetzung nach Anspruch 1, wobei die Zusammensetzung von 1 Gew.-% bis 15 Gew.-%, als Alternative von 5 Gew.-% bis 10 Gew.-%, als Alternative von 0,1 Gew.-% bis 10 Gew.-%, und als Alternative von 10 Gew.-% bis 20 Gew.-% einer Sulfopropylacrylatverbindung bezogen auf die Gesamtzusammensetzung umfasst.

10. Kit, umfassend:

   a) eine erste Zusammensetzung, umfassend von 0,1 Gew.-% bis 20 Gew.-% einer Sulfopropylacrylatverbindung bezogen auf die Gesamtzusammensetzung, wobei die Sulfopropylacrylatverbindung ein Molekulargewicht von weniger als 500 Gramm/Mol aufweist; und einen dermatologisch akzeptablen Träger; wobei die Zusammensetzung von 60 Gew.-% bis 99,9 Gew.-% eines dermatologisch akzeptablen Trägers bezogen auf die Gesamtzusammensetzung umfasst; und
   b) eine zweite Zusammensetzung, die einen Initiator umfasst;

   wobei die erste Zusammensetzung und die zweite Zusammensetzung getrennt gepackt sind.

11. Kit nach Anspruch 10, wobei die zweite Zusammensetzung von 0,001 Gew.-% bis 5 Gew.-%, als Alternative von 0,01 Gew.-% bis 1 Gew.-%, als Alternative von 0,1 Gew.-% bis 1 Gew.-% eines Initiators bezogen auf die Gesamtzusammensetzung umfasst.

12. Kit nach einem der Ansprüche 10 und 11, wobei der Initiator ausgewählt ist aus: Peroxidisulfaten, Peroxiden, Persäuren, Phosphaten, Manganaten, Boraten, bis-Alkylamidinen, Sulfiten, Peroxyestern, bis-Cyanocarbonsäuren, Alphaaminoessigsäuren und Mischungen davon.

13. Kit nach einem der Ansprüche 10 bis 11, wobei die zweite Zusammensetzung einen pH-Wert über 7,0, als Alternative von 7,0 bis 12,0, als Alternative von 8,0 bis 12,0, als Alternative von 9,0 bis 11,0 aufweist.

14. Kit nach einem der Ansprüche 10 bis 13, wobei das Kit ferner eine Reduktionszusammensetzung umfasst, wobei die Reduktionszusammensetzung von 1 Gew.-% bis 12 Gew.-%, als Alternative von 4 Gew.-% bis 10 Gew.-%, als Alternative von 8 Gew.-% bis 10 Gew.-% eines Reduktionsmittels bezogen auf die Gesamtzusammensetzung umfasst.

15. Kit nach Anspruch 14, wobei das Reduktionsmittel ausgewählt ist aus Natriumthioglycolat, wasserfreiem Natriumthiosulfat, pulverförmigem Natriumdisulfit, Thioharnstoff, Ammoniumsulfit, Thioglycolsäure, Thiomilchsäure, Ammoniumthiolactat, Glycerylmonothioglycolat, Ammoniumthioglycolat, Thioglycerol, 2,5-Dihydroxybenzoesäure, Diammoniumdithioglycolat, Strontiumthioglycolat, Calciumthioglycolat, Zinkformosulfoxylat, Isooctylthioglycolat, Di-cystein, Monoethanolaminthioglycolat, Phosphinen und Mischungen davon.

**Revendications**

1. Composition pour soins capillaires comprenant de 0,1 % à 20 % en poids de la composition totale, d'un composé sulfopropylacrylate, dans laquelle le composé sulfopropylacrylate a une masse moléculaire inférieure à 500 grammes/mole ; et un véhicule acceptable du point de vue dermatologique ; où la composition comprend de 60 % à 99,9 % en poids de la composition totale d'un véhicule acceptable du point de vue dermatologique.

2. Composition selon la revendication 1, comprenant en outre de 0,1 % à 20 % en poids de la composition totale, d'au moins un composé supplémentaire choisi dans le groupe constitué d'un monomère éthylénique, un agent de réticulation et leurs mélanges, dans laquelle le composé supplémentaire a une masse moléculaire inférieure à 500 g/mole.

3. Composition selon la revendication 1, comprenant en outre de 0,1 % à 20 % en poids de la composition totale, chacun de monomère éthylénique et d'un agent de réticulation, dans laquelle le monomère éthylénique et l'agent de réticulation ont chacun une masse moléculaire inférieure à 500 g/mole.

4. Composition selon la revendication 2, dans laquelle le monomère éthylénique est choisi dans le groupe constitué d'acide mésaconique, acide tert-2-penténoïque, acide tiglique, esters d'acide tiglique, acide furan-3-acrylique, acide 2-acrylamido-2-méthyl-1-propane sulfonique, acide maléamique, acide 3-aminocrotonique, esters d'acide crotonique, anhydride itaconique, triméthylsilylacrylate, poly(éthylèneglycol)acrylates, N-vinylacétamide, acide 2-acétamidoacrylique, acide vinylsulfonique, tétrahydrofurfurylacrylate, N-méthyl-N-vinylacétamide, vinylpropionate, vinylanisole, vinylcrotonate, méthyl 3-hydroxy-2-méthylènebutyrate et leurs mélanges.

5. Composition selon la revendication 2, dans laquelle l'agent de réticulation est choisi dans le groupe constitué de 1,4-bisacryloylpipérazine, méthylène-bisacrylamide, éthylènebisacrylamide, divinylbenzène, poly-éthylèneglycol di(méth)acrylate, éthylène glycol di(méth)acrylate, 1,3-butanediol di(méth)acrylate, 1,4-butanediol di(méth)acrylate, néopentyle glycol di(méth)acrylate et leurs mélanges.

6. Composition selon la revendication 2, dans laquelle le deuxième composé est un monomère éthylénique, et dans laquelle le rapport du pourcentage en poids du composé sulfopropylacrylate sur le pourcentage en poids du monomère éthylénique va de 1:12 à 12:1.

7. Composition selon la revendication 2, dans laquelle le deuxième composé est un agent de réticulation et dans laquelle le rapport du pourcentage en poids du composé sulfopropylacrylate sur le pourcentage en poids de l'agent réticulant va de 50:1 à 10:1.

8. Composition selon la revendication 1, dans laquelle le pH de la composition est 7,0 et plus bas.

9. Composition selon la revendication 1, où la composition comprend de 1 % à 15 %, en variante, de 5 % à 10 %, en variante, de 0,1 % à 10 % et, en variante, de 10 % à 20 % en poids de la composition totale d'un composé sulfopropylacrylate.

10. Trousse comprenant :

a) une première composition comprenant de 0,1 % à 20 % en poids de la composition totale, d'un composé sulfopropylacrylate, dans laquelle le composé sulfopropylacrylate a une masse moléculaire inférieure à 500 grammes/mole ; et un véhicule acceptable du point de vue dermatologique ; dans laquelle la composition comprend de 60 % à 99,9 % en poids de la composition totale d'un véhicule acceptable du point de vue dermatologique ; et

b) une deuxième composition comprenant un inducteur ;

dans laquelle la première composition et la deuxième composition sont conditionnées séparément.

**EP 2 114 360 B1**

11. Trousse selon la revendication 10, dans laquelle la deuxième composition comprend de 0,001 % à 5 %, en variante, de 0,01 % à 1 %, en variante, de 0,1 % à 1 % en poids de la composition totale d'un inducteur.

12. Trousse selon l'une quelconque des revendications 10 et 11, dans laquelle l'inducteur est choisi parmi : des peroxydisulfates, des peroxydes, des peracides, des phosphates, des manganates, des borates, des bis-alkylamidines, des sulfites, des peroxyesters, des acides bis-cyanocarboxyliques, des acides alpha-amino acétique et leurs mélanges.

13. Trousse selon l'une quelconque des revendications 10 à 11, dans laquelle la deuxième composition a un pH supérieur à 7,0, en variante, allant de 7,0 à 12,0, en variante, allant de 8,0 à 12,0, en variante, allant de 9,0 à 11,0.

14. Trousse selon l'une quelconque des revendications 10 à 13, où la trousse comprend en outre une composition réductrice, dans laquelle la composition réductrice comprend de 1 % à 12 %, en variante, de 4 % à 10 %, en variante, de 8 % à 10 % en poids de la composition totale d'un agent réducteur.

15. Trousse selon la revendication 14, dans laquelle l'agent réducteur est choisi parmi le thioglycolate de sodium, le thiosulfate de sodium anhydre, le métabisulfite de sodium pulvérulent, la thio-urée, le sulfite d'ammonium, l'acide thioglycolique, l'acide thiolactique, le thiolactate d'ammonium, le monothioglycolate de glycéryle, le thioglycolate d'ammonium, le thioglycérol, l'acide 2,5-dihydroxybenzoïque, le dithioglycolate de diammonium, le thioglycolate de strontium, le thioglycolate de calcium, le formosulfoxylate de zinc, le thioglycolate d'iso-octyle, la dl-cystéine, le thioglycolate de monoéthanolamine, des phosphines et leurs mélanges

**16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- International Cosmetic Ingredient Dictionary. 2002

- CTFA Cosmetic Ingredient Handbook. 2004